# EUROPEAN PATENT SPECIFICATION

(11) **EP 0 404 497 B1**
(45) Date of publication and mention of the grant of the patent: **01.06.1994**
(21) Application number: 90306647.0
(22) Date of filing: 19.06.1990
(51) Int. Cl.: C07C 45/54, C07C 49/597, C07C 49/603, C07C 49/203

(54) **A process for producing an alpha,beta-unsaturated carbonyl compound**
Verfahren zur Herstellung einer alpha,beta-ungesättigten Carbonylverbindung
Procédé pour la fabrication d'un composé carbonyle alpha,bêta-insaturé

(30) Priority: 22.06.1989 JP 160428/89
(43) Date of publication of application: 27.12.1990
(73) Proprietor: NIPPON ZEON CO., LTD., Tokyo 109 (JP)
(72) Inventor: Matsuoka, Rikitaro, Yokohama-shi (JP); Watanabe, Kiyoshi, Yokohama-shi (JP)
(74) Representative: Harrison, David Christopher

(56) References cited:
- PATENT ABSTRACTS OF JAPAN, vol 9, no. 160 (C-289)(1883), July 4, 1985 & JP-A-6036435
- PATENT ABSTRACTS OF JAPAN, vol 8, no 178 (C-238)(1615), August 16, 1984, & JP-A-5973536
- CHEMISTRY LETTERS, vol 1984 ; J. TSUJI et al, "Enone formation from allyl beta-keto esters, alkenyl allyl carbonates, silyl enol ethers, and enol aceates by the phosphin-free palladium catalyst"
- SYNTHESIS, vol. 2, 1987, no. 11, Stuttgart, DE; I. MINAMI et al, "New methods for the synthesis of alpha,beta-unsaturated ketones, aldehydes, and nitriles by the palladium-catalyzed reactions of allyl beta-oxo esters, allyl 1-alkenyl carbonates, and allyl alpha-cyano esters"
- CHEMISTRY LETTERS, vol 10, 1984, Tokyo, JP ; J. TSUJI et al, "Synthesis of gamma,delta-unsaturated ketones by the intramolecular decarboxylative allylation of allyl beta-keto carboxylates and alkenyl allyl carbonates catalyzed by molybdenum, nickel, and rhodium complexes"

## Description

This invention relates to a novel process for producing an α,β-unsaturated carbonyl compound.

### RELATED ART STATEMENT

α,β-unsaturated carbonyl compounds such as 2-cyclopentenone, 2-cyclohexenone, 2-cyclododecenone, etc. are useful chemical substances in the fields of perfumes, medicines, chemicals, etc. As a process for synthesizing such an unsaturated carbonyl compound, there has been reported a process which comprises treating an alkenyl ester and an allyl type carbonic ester with a platinum group metal compound catalyst in the presence of an organotin alkoxide (Jap. Pat. Appln. Kokai (Laid-Open) No. 60-36435, and Chemistry Letters 1133-1136 (1984)). However, the organotin alkoxide used as indispensable component in this process is difficult to handle and it is insufficient in safety from the viewpoint of industrial hygiene and contamination of the organotin alkoxide to a final product.

### SUMMARY OF THE INVENTION

In order to solve the above-stated problem, the present inventors have earnestly investigated. Consequently they unexpectedly found that the above reaction can be allowed to proceed by the use of a platinum group metal compound catalyst alone without using an organotin alkoxide which is used as an indispensable component in the above report. On the basis of the above finding, the present invention has been accomplished.

Thus, according to the present invention, there is provided a process for producing an α,β-unsaturated carbonyl compound of the general formula [II] shown below, comprising bringing an alkenyl ester of the general formula [I] shown below into contact with an allyl type carbonic ester in the presence of a platinum group metal compound catalyst, which process is superior to that of the prior art in operations and safety and can give an α,β-unsaturated carbonyl compound in high yield. 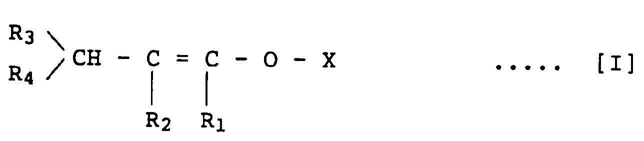 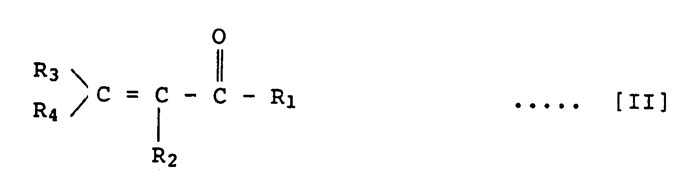
(wherein R₁, R₂, R₃ and R₄ are independently hydrogen atoms or hydrocarbon residues, and R₁, R₂, R₃ and R₄ may be linear or may form one or more rings when taken together in arbitrary combinations; and X is an acyl group.)

### DETAILED DESCRIPTION OF PREFERRED EMBODIMENTS

In the present invention, an alkenyl ester of the above general formula [I] is used as a first starting material. In the general formula [I], R₁ is a hydrogen atom or a hydrocarbon residue. The hydrocarbon residue includes an alkyl group such as a methyl, ethyl, propyl, pentyl group, etc.; an alkylene group which are bonded to R₂, R₃ or R₄ to form a ring such as a cyclopentane, cyclohexane, cyclododecane ring or the like; and an aryl group such as a phenyl, trityl group, etc. R₂, R₃ and R₄ are independently hydrogen atoms or the same alkyl, alkylene or aryl group as R₁. X is an acyl group. The above substituents R₁, R₂, R₃ and R₄ may be taken together in arbitrary combinations to form one or more rings.

Specific examples of such a compound are 1-cyclopentenyl acetate, 1-cyclohexenyl acetate, 6-methyl-1-cyclopentenyl acetate, 6-ethyl-1-cyclopentenyl acetate, 1-cycloheptenyl acetate, 1-cyclododecenyl acetate, 1-cyclopentenyl propionate, 1-cyclopentenyl butylate, 1-cyclopentenyl benzoate, 1-phenyl-1-butenyl acetate, 1-propenyl acetate, 1-hexenyl acetate, 3-methyl-1-butenyl acetate, 3-phenyl-1-propenyl acetate, etc. Of these, cycloalkenyl esters are preferable. Of esters, cyclopentenyl esters are particularly preferable because they permit progress of the reaction with ease and can give a desired product in high yield.

These compounds may be synthesized by a conventional method. For example, 1-cyclopentenyl acetate can easily be synthesized by such a method that comprises reacting cyclopentanone with isopropenyl acetate in the presence of an acid, or reacting cyclopentanone with acetic anhydride.

The allyl type carbonic ester used as a second starting material is a carbonic ester having at least one allyl or its allied residue and it is usually a compound of the general formula [III]: 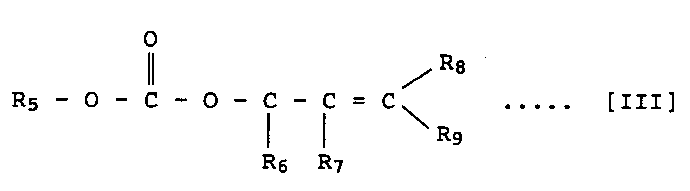
wherein R₅ is a hydrocarbon residue, and R₆, R₇, R₈ and R₉ are independently hydrogen atoms or hydrocarbon residues.

The hydrocarbon residues include, for example, alkyl groups having 1 to 8 carbon atoms (e.g. methyl, ethyl group, etc.) and alkenyl groups having 2 to 8 carbon atoms.

Specific examples of such a compound are allyl methyl carbonate, allyl ethyl carbonate, allyl propyl carbonate, allyl butyl carbonate, allyl pentyl carbonate, crotyl methyl carbonate, ethyl methallyl carbonate, diallyl carbonate, etc. Of these, there are preferably used allyl esters, crotyl esters and methallyl esters, in which R₅ is a lower alkyl group having 4 or less carbon atoms.

In the present invention, a platinum group metal compound catalyst is used in the reaction. The platinum group metal compound catalyst is a platinum group metal compound per se or it is composed of a platinum group metal compound and a ligand.

The platinum group metal compound used is a salt or complex of palladium, platinum, rhodium, iridium or ruthenium. Specific examples thereof are tris(dibenzylideneacetone)dipalladium (0), tris(tribenzylideneacetylacetone)tripalladium (0), palladium acetate, palladium propionate, palladium butyrate, palladium benzoate, palladium acetylacetonate, palladium nitrate, palladium sulfate, palladium chloride platinum [II] acetate, platinum acetylacetonate, etc. In the case where an inorganic strong acid salt of these compounds is used, it is preferable to place together therewith a base such as potassium acetate, sodium alkoxides, tertiary amines or the like. Of platinum group metals, palladium is particularly preferable from the viewpoint of reactivity, and employment of a zerovalent olefin complex or a divalent organic compound is particularly preferable.

The ligand used is a monodentate or multidentate electron donor compound having, as a ligand atom, an element of Group V of the periodic table, i.e., nitrogen, phosphorus, arsenic or antimony. Specific examples of the ligand are nitrogen-containing compounds such as pyridine, quinoline, trimethylamine, triethylamine, tributylamine, α,α-dipyridine, 1,10-phenanthroline, N,N,N′,N′-tetramethylethylenediamine, etc.; phosphorus-containing compounds such as triethylphosphine, tri-n-butylphosphine, tri-n-dodecylphosphine, triphenylphosphine, tri-o-tolylphosphine, tri-p-biphenylphosphine, tri-o-methoxyphenylphosphine, phenyldiphenoxyphosphine, triethyl phosphite, tri-n-butyl phosphite, tri-n-hexyl phosphite, triphenyl phosphite, tri-o-tolyl phosphite, triphenyl thiophopshite, α,β-ethylenedi(diphenyl)phosphine, α,β-ethylenedi(dibutyl)phosphine, α,γ-propylenedi(diphenyl)phosphine, etc.; arsenic-containing compounds such as triethylarsine, tributylarsine, triphenylarsine, etc.; and antimony-containing compounds such as tripropylstibine, triphenylstibine, etc. Of these, the nitrogen-containing compounds and the phosphorus-containing compounds are preferable from the viewpoint of, for example, the activity, selectivity and economical benefit of reaction.

Although such a ligand is not always indispensable as a constituent of the catalyst, it can greatly improve the stability of the catalyst when used in a suitable amount.

The amount of the ligand used is varied depending on the kind thereof, but it is usually 2.5 moles or less, preferably 0.1 to 2 moles, per mole of the metal compound.

The amount of the catalyst in the present invention is properly chosen. Said amount is usually in such a proportion that the amount of the platinum group metal compound is 0.01 to 10 moles, preferably 0.1 to 5 moles, per 100 moles of the alkenyl ester. Although the platinum group metal compound and the ligand may be previously reacted with each other, the catalyst is usually prepared by bringing these components into contact with each other in a reaction system.

The reaction of the present invention is carried out by heating the above two starting materials in the presence of the catalyst. For example, a reaction formula in the case of using 1-cyclopentenyl acetate and allyl methyl carbonate is as follows:

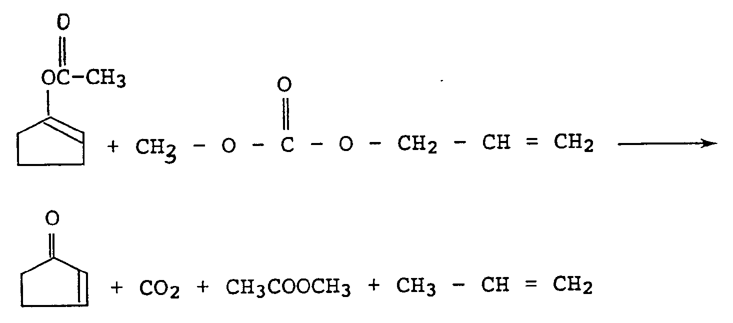

As to the proportions of the starting materials to be used, the allyl type carbonic ester is usually used in an amount of 0.8 to 5 moles, preferably 1 to 3 moles, per mole of the alkenyl ester. The reaction temperature is usually 50°C or higher, preferably 55°C to 150°C, and the reaction time is usually 10 minutes to 20 hours.

A diluent can be present in the reaction. Specific examples of the diluent are nitriles such as acetonitrile, propionitrile, butyronitrile, benzonitrile, etc.; sulfoxides such as dimethyl sulfoxide, diethyl sulfoxide, etc.; amides such as dimethylformamide, diethylformamide, dimethylacetamide, dimethylpropioamide, N-methylpyrrolidone, etc.; ethers such as tetrahydrofuran, dioxane, dibutyl ether, ethylene glycol dimethyl ether, etc.; ketones such as acetone, methyl ethyl ketone, methyl isobutyl ketone, cyclohexanone, etc.; esters such as methyl acetate, ethyl acetate, propyl acetate, ethyl propionate, etc.; alcohols such as ethanol, propanol, tert-butanol, ethylene glycol, diethylene glycol monoethyl ether, etc.; and hydrocarbons such as n-hexane, cyclohexane, benzene, toluene, xylene, etc. Of these, aprotic polar solvents, in particular, the nitriles and the sulfoxides are preferable.

The above-exemplified diluents are usually used in such a proportion that the concentration of the starting materials becomes 1 to 50% by weight. The diluent can improve an activity and selectivity of the reaction and the stability of the catalyst.

After completion of the reaction, a desired product is separated from the reaction mixture by a conventional method, whereby there can be obtained an α,β-unsaturated carbonyl compound (i.e. an α,β-unsaturated ketone or an α,β-unsaturated aldehyde) of high purity. Such an unsaturated carbonyl compound is used as an intermediate for synthesis of various useful compounds, in particular, as an intermediate for perfumes, medicines, etc.

The present invention is more specifically illustrated with the following examples.

### Example 1

Into a reactor were charged 0.1 mole of 1-cyclopentenyl acetate, 0.12 mole of allyl methyl carbonate ad 50 ml of acetonitrile, and the mixture heated up to the boiling point of the solvent with stirring; and 0.001 mole of palladium acetate was added. Then, the reaction was carried out with refluxing under nitrogen for 2 hours. The reaction mixture was analyzed by gas chromatography to find tat 2-cyclopenten-1-one (hereinafter abbreviated to CPE) had been produced in 93% yield. Such a compound was identified by IR, NMR and MS spectroscopy.

### Example 2

Reaction was carried out in the same manner as in Example 1, except for adding 0.001 mole of triphenylphosphine in addition to palladium acetate. Consequently, the yield of CPE was 94%.

### Example 3

Reaction was carried out in the same manner as in Example 1, except for using 0.001 mole of tris(dibenzylideneacetone)dipalladium (0) in place of palladium acetate. Consequently, the yield of CPE was 92%.

### Example 4

Reaction was carried out according to Example 1, except for using 50 ml of dimethyl sulfoxide in place of acetonitrile. Consequently, the yield of CPE was 93%.

### Example 5

Reaction was carried out according to Example 4, except for using 1-cyclohexenyl acetate as a starting material. Consequently, 2-cyclohexen-1-one was obtained in 42% yield.

### Example 6

Reaction was carried out according to Example 4, except for using 4-heptenyl acetate as a starting material. Consequently, 2-hepten-4-one was obtained in 26% yield.

## Claims

1. A process for producing an α,β-unsaturated carbonyl compound represented by the general formula [II]: 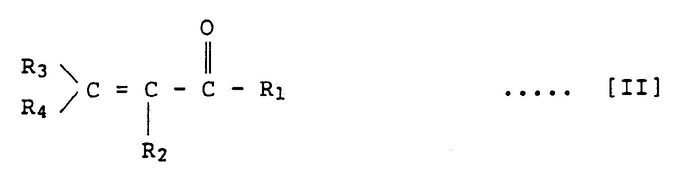 wherein R₁, R₂, R₃ and R₄ are independently hydrogen atoms or chain hydrocarbon residues, and R₁, R₂, R₃ and R₄ may be linear or may form one or more rings when taken together in arbitrary combination which comprises bringing an alkenyl ester represented by the general formula [I]: 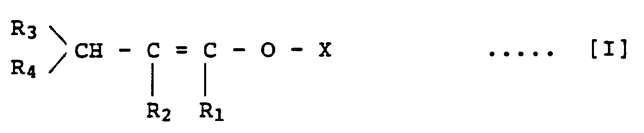 wherein R₁, R₂, R₃ and R₄ have the same meanings as those defined above, and X is an acyl group in the presence of a platinum group metal compound catalyst into contact with a carbonic ester of the general formula [III]: 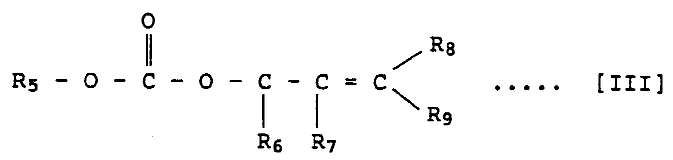 wherein R₅ is a hydrocarbon residue, and R₆, R₇, R₈ and R₉ are independently hydrogen atoms or hydrocarbon residues and wherein the reaction is carried out in the absence of an organotin alkoxide.

2. The process according to Claim 1, wherein R₁, R₂, R₃ and R₄ are independently hydrogen atoms or hydrocarbon groups selected from alkyl groups, alkenyl groups or aryl groups.

3. The process according to Claim 1, wherein the alkenyl ester represented by the general formula [I] is a cycloalkenyl ester.

4. The process according to Claim 3, wherein the cycloalkenyl ester is a cyclopentenyl ester.

5. The process according to Claim 1, wherein the carbonic ester is a lower alkyl ester of allyl carbonate, crotyl carbonate or methally carbonate.

6. The process according to claim 1, wherein the catalyst is a platinum group metal compound or a catalyst composed of a platinum group metal compound and a ligand.

7. The process according to Claim 6 wherein the platinum group metal compound is a salt or a complex of a platinum group metal selected from palladium, platinum, rhodium, iridium or ruthenium.

8. The process according to Claim 7 wherein the platinum group metal is palladium.

9. The process according to Claim 7 wherein the platinum group metal compound is a zerovalent olefin complex or a divalent organic compound of palladium.

10. The process according to Claim 6 wherein the ligand is a monodentate or multidentate electron donor compound having, as a ligand atom, an element of Group V of the periodic table.

11. The process according to Claim 10 wherein said element is N, P, As or Sb.

12. The process according to Claim 6 wherein the ligand is used in amount of 2.5 moles or less per mole of the metal compound.

13. The process according to Claim 1 wherein the catalyst is used in amount selected from 0.01 to 10 moles per 100 moles of the alkenyl ester.

14. The process according to Claim 1 wherein the carbonic ester is used in amount of from 0.8 to 5 moles per mole of the alkenyl ester.

15. The process according to Claim 1 wherein the contact between the alkenyl ester with the carbonic ester is carried out at a temperature of 50°C or more for a period of from 10 minutes to 20 hours.

16. The process according to Claim 1 wherein the contact is carried out in the presence of a diluent.

17. The process according to Claim 16 wherein the diluent is one selected from nitriles, sulfoxides, amides, ethers, ketones, esters, alcohols and hydrocarbons.

18. The process according to Claim 16 wherein the diluent is an aprotic polar solvent.

19. The process according to Claim 16 wherein the diluent is used in such an amount that the concentration of the esters becomes 1 to 50% by weight.

## Patentansprüche

1. Verfahren zur Herstellung einer α,β-ungesättigten Carbonylverbindung der allgemeinen Formel [II] 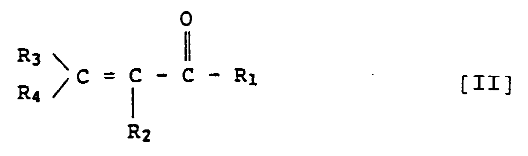 in der R₁, R₂, R₃ und R₄ unabhängig voneinander Wasserstoffatome oder Reste von Kohlenwasserstoffketten sind und R₁, R₂, R₃ und R₄ linear sein können oder in beliebigen Kombinationen miteinander einen oder mehrere Ringe bilden können, umfassend das Inkontaktbringen eines Alkenylesters der allgemeinen Formel [I] 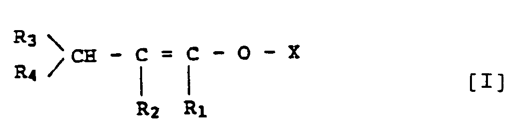 in der R₁, R₂, R₃ und R₄ die vorstehend definierten Bedeutungen haben und X ein Acylrest ist, in Gegenwart einer Verbindung eines Platinmetalls als Katalysator mit einem Kohlensäureester der allgemeinen Formel [III] 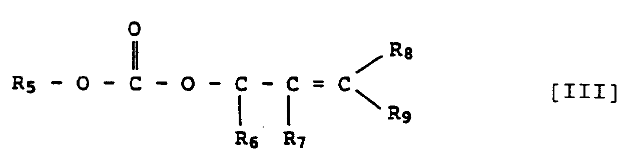 in der R₅ ein Kohlenwasserstoffrest ist und R₆, R₇, R₈ und R₉ unabhängig voneinander Wasserstoffatome oder Kohlenwasserstoffreste sind, und wobei die Umsetzung in Abwesenheit eines Organozinnalkoxids durchgeführt wird.

2. Verfahren nach Anspruch 1, in dem R₁, R₂, R₃ und R₄ unabhängig voneinander Wasserstoffatome oder Kohlenwasserstoffreste sind, gewählt aus Alkyl-, Alkenyl- oder Arylresten.

3. Verfahren nach Anspruch 1, in dem der Alkenylester der allgemeinen Formel [I] ein Cycloalkenylester ist.

4. Verfahren nach Anspruch 3, in dem der Cycloalkenylester ein Cyclopentenylester ist.

5. Verfahren nach Anspruch 1, in dem der Kohlensäureester ein Niederalkylester der Allylkohlensäure, Crotylkohlensäure oder Methallylkohlensäure ist.

6. Verfahren nach Anspruch 1, in dem der Katalysator eine Verbindung eines Platinmetalls oder ein Katalysator ist, der aus einer Verbindung eines Platinmetalls und einem Liganden besteht.

7. Verfahren nach Anspruch 6, in dem die Verbindung eines Platinmetalls ein Salz oder ein Komplex eines Platinmetalls gewählt aus Palladium, Platin, Rhodium, Iridium oder Ruthenium ist.

8. Verfahren nach Anspruch 7, in dem das Platinmetall Palladium ist.

9. Verfahren nach Anspruch 7, in dem die Verbindung eines Platinmetalls ein nullwertiger Olefinkomplex oder eine zweiwertige organische Verbindung des Palladiums ist.

10. Verfahren nach Anspruch 6, in dem der Ligand eine einzähnige oder mehrzähnige Elektronendonorverbindung ist, die als Ligandenatom ein Element der Gruppe V des Periodensystems aufweist.

11. Verfahren nach Anspruch 10, in dem das Element Stickstoff, Phosphor, Arsen oder Antimon ist.

12. Verfahren nach Anspruch 6, in dem der Ligand in einer Menge von 2,5 Mol oder weniger pro Mol der Metallverbindung verwendet wird.

13. Verfahren nach Anspruch 1, in dem der Katalysator in einer Menge von 0,01 bis 10 Mol pro 100 Mol des Alkenylesters verwendet wird.

14. Verfahren nach Anspruch 1, in dem der Kohlensäureester in einer Menge von 0,8 bis 5 Mol pro Mol des Alkenylesters verwendet wird.

15. Verfahren nach Anspruch 1, in dem der Alkenylester 10 Minuten bis 20 Stunden bei einer Temperatur von 50°C oder mehr mit dem Kohlensäureester in Kontakt gebracht wird.

16. Verfahren nach Anspruch 1, in dem der Kontakt in Gegenwart eines Verdünnungsmittels durchgeführt wird.

17. Verfahren nach Anspruch 16, in dem das Verdünnungsmittel ausgewählt ist aus Nitrilen, Sulfoxiden, Amiden, Ethern, Ketonen, Estern, Alkoholen und Kohlenwasserstoffen.

18. Verfahren nach Anspruch 16, in dem das Verdünnungsmittel ein polar aprotisches Lösungsmittel ist.

19. Verfahren nach Anspruch 16, in dem das Verdünnungsmittel in einer solchen Menge verwendet wird, daß die Konzentration der Ester 1 bis 50 Gew.-% wird.

## Revendications

1. Procédé de préparation d'un composé carbonylé α,β-insaturé, représenté par la formule générale [II] : 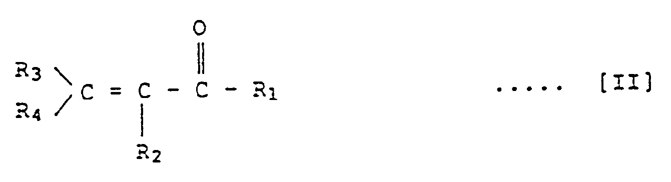 dans laquelle R₁, R₂, R₃ et R₄ représentent indépendamment des atomes d'hydrogène ou des groupes hydrocarbonés, R₁, R₂, R₃ et R₄ pouvant être linéaires ou former un ou plusieurs cycles si on les considère conjointement en une combinaison arbitraire,
lequel procédé comporte le fait de mettre un ester alcénylique représenté par la formule générale [I] : 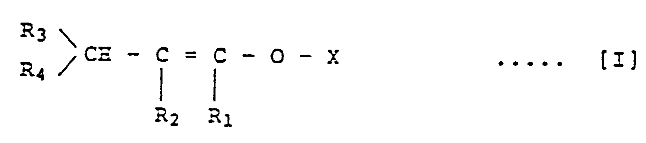 dans laquelle R₁, R₂, R₃ et R₄ ont les mêmes significations que celles indiquées ci-dessus, et X représente un groupe acyle, en présence d'un catalyseur à base d'un composé d'un métal du groupe du platine, en contact avec un ester carbonate de formule générale [III] : 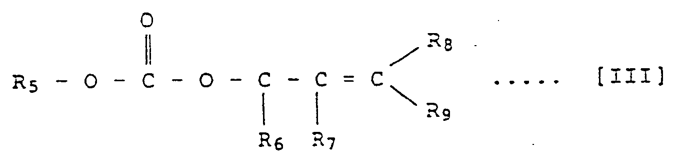 dans laquelle R₅ représente un groupe hydrocarboné et R₆, R₇, R₈ et R₉ représentent indépendamment des atomes d'hydrogène ou des groupes hydrocarbonés,
la réaction étant effectuée en l'absence d'un alcoxyde d'organo-étain.

2. Procédé conforme à la revendication 1, dans lequel R₁, R₂, R₃ et R₄ représentent indépendamment des atomes d'hydrogène ou des groupes hydrocarbonés choisis parmi les groupes alkyle, les groupes alcényle et les groupes aryle.

3. Procédé conforme à la revendication 1, dans lequel l'ester alcénylique représenté par la formule générale [I] est un ester cycloalcénylique.

4. Procédé conforme à la revendication 3, dans lequel l'ester cycloalcénylique est un ester de cyclopentényle.

5. Procédé conforme à la revendication 1, dans lequel l'ester carbonate est un carbonate d'alkyle inférieur et d'allyle, de crotyle ou de méthallyle.

6. Procédé conforme à la revendication 1, dans lequel le catalyseur est un composé d'un métal du groupe du platine, ou bien un catalyseur constitué d'un composé d'un métal du groupe du platine et d'un ligand.

7. Procédé conforme à la revendication 6, dans lequel le composé d'un métal du groupe du platine est un sel ou un complexe d'un métal du groupe du platine, choisi parmi le palladium, le platine, le rhodium, l'iridium et le ruthénium.

8. Procédé conforme à la revendication 7, dans lequel le métal du groupe du platine est le palladium.

9. Procédé conforme à la revendication 7, dans lequel le composé d'un métal du groupe du platine est un complexe d'oléfine et de métal zérovalent ou un composé organique de palladium divalent.

10. Procédé conforme à la revendication 6, dans lequel le ligand est un composé donneur d'électrons, monodenté ou polydenté, et comportant, en tant qu'atome de liaison, un élément du Groupe V du Tableau Périodique.

11. Procédé conforme à la revendication 10, dans lequel ledit élément est N, P, As ou Sb.

12. Procédé conforme à la revendication 6, dans lequel on utilise le ligand à raison de 2,5 moles ou moins par mole de composé de métal.

13. Procédé conforme à la revendication 1, dans lequel on utilise le catalyseur en une proportion choisie dans l'intervalle allant de 0,01 à 10 moles pour 100 moles d'ester alcénylique.

14. Procédé conforme à la revendication 1, dans lequel on utilise l'ester carbonate à raison de 0,8 à 5 moles par mole d'ester alcénylique.

15. Procédé conforme à la revendication 1, dans lequel on laisse l'ester alcénylique en contact avec l'ester carbonate pendant un laps de temps de 10 minutes à 20 heures, à une température égale ou supérieure à 50°C.

16. Procédé conforme à la revendication 1, dans lequel on réalise la mise en contact en présence d'un diluant.

17. Procédé conforme à la revendication 16, dans lequel le diluant est choisi parmi les nitriles, sulfoxydes, amides, éthers, cétones, esters, alcools et hydrocarbures.

18. Procédé conforme à la revendication 16, dans lequel le diluant est un solvant polaire aprotique.

19. Procédé conforme à la revendication 16, dans lequel on utilise le diluant en une quantité telle que la concentration des esters vaille de 1 à 50 % en poids.
